# EUROPEAN PATENT APPLICATION

(11) **EP 3 998 472 A1**
(43) Date of publication of application: **18.05.2022**
(21) Application number: 20837850.5
(22) Date of filing: 10.07.2020
(51) Int. Cl.: G01N 21/78, G01N 33/00, B01J 23/42, B01J 23/44, G01N 31/10

(54) **HYDROGEN SENSOR AND MANUFACTURING METHOD THEREFOR**

(30) Priority: 10.07.2019 KR 20190083060
(71) Applicant: Daehyunst Co., Ltd, Hwaseong-si, Gyeonggi-do 18578 (KR)
(72) Inventor: SEO, Hyung Tak, Seoul 06544 (KR); HAN, Seung Ik, Gyeonggi-do 16323 (KR); LEE, Yeong An, Gyeongsangnam-do 53042 (KR)
(74) Representative: Laine IP Oy
(86) International application number: PCT/KR2020/009078
(87) International publication number: WO 2021/006689

(57) **Abstract**

Disclosed is a hydrogen sensor. The hydrogen sensor includes a substrate; a color changeable layer disposed on the substrate and made of an oxide semiconductor material, wherein when the oxide semiconductor material reacts with hydrogen ions or hydrogen atoms, a color thereof changes; a catalyst layer disposed on a surface of the color changeable layer and made of a catalyst material, wherein the catalyst material dissociates hydrogen molecules (H2) into hydrogen atoms (H) or hydrogen ions (H⁺); and a protective layer disposed to cover a surface of the catalyst layer and an exposed surface of the color changeable layer, and made of a polymer material, wherein the polymer material allows hydrogen molecules to pass therethrough but blocks water molecules.

## Description

### FIELD

The present disclosure relates to a hydrogen sensor capable of sensing hydrogen based on color change of a metal oxide and a method for manufacturing the same.

### DESCRIPTION OF RELATED ART

Hydrogen gas (H₂) is a source of renewable clean energy with a wide range of applications to a chemical field, fuel cell, automobile fuel, and rocket engines. Hydrogen gas as a fuel is in the spotlight as a future energy because it burns completely with a high heat of combustion of 142 kJ/g. However, hydrogen gas is highly volatile, explosive and flammable, and is dangerous when a hydrogen concentration exceeds a critical value.

Hydrogen gas is a flammable gas without color, odor, and taste, and thus cannot be sensed by the human senses. Therefore, in order to safely use hydrogen, a hydrogen sensor is essential. Various types of hydrogen sensors have been reported. Among them, interest in electrical sensors and gasochromic sensors is increasing. However, although the electrical sensor has a high sensing ability, it has a problem in that a malfunction occurs due to electromagnetic noise or use environment thereof is limited.

Hydrogen has recently been developed as an energy source in various industries. In this case, it is necessary to develop a technology to detect hydrogen dissolved in liquid as well as hydrogen in the atmosphere.

### DISCLOSURE

### TECHNICAL PURPOSES

One purpose of the present disclosure is to provide a hydrogen sensor capable of simultaneously sensing hydrogen dissolved in liquid as well as gaseous hydrogen in the atmosphere.

Another purpose of the present disclosure is to provide a method for manufacturing the hydrogen sensor.

### TECHNICAL SOLUTIONS

One aspect of the present disclosure provides a hydrogen sensor comprising: a substrate; a color changeable layer disposed on the substrate and made of an oxide semiconductor material, wherein when the oxide semiconductor material reacts with hydrogen ions or hydrogen atoms, a color thereof changes; a catalyst layer disposed on a surface of the color changeable layer and made of a catalyst material, wherein the catalyst material dissociates hydrogen molecules (H₂) into hydrogen atoms (H) or hydrogen ions (H⁺); and a protective layer disposed to cover a surface of the catalyst layer and an exposed surface of the color changeable layer, and made of a polymer material, wherein the polymer material allows hydrogen molecules to pass therethrough but blocks water molecules.

In one embodiment, the catalyst layer is made of palladium (Pd), platinum (Pt) or an alloy containing at least one thereof.

In one embodiment, the catalyst layer is formed to have a thickness in a range of 4 to 4.5nm.

In one embodiment, the color changeable layer and the catalyst layer are sealed with the substrate and the protective layer.

In one embodiment, the protective layer is made of PVB (polyvinyl butyral).

In one embodiment, the protective layer is formed to have a thickness in a range of 4.4 to 5.4µm.

Another aspect of the present disclosure provides a method for manufacturing a hydrogen sensor, the method comprising: depositing tungsten oxide on a substrate to form a color changeable layer; depositing palladium or platinum on the color changeable layer to form a catalyst layer; an performing a cycle 4 to 6 times to form a protective layer covering a surface of the catalyst layer and an exposed surface of the color changeable layer, wherein the cycle is composed of a first step of forming a PVB film on the catalyst layer using a spin coating process of a PVB (polyvinyl butyral) solution and a second step of spin-coating ultrapure water on the PVB film.

In one embodiment of the method, the protective layer is formed to have a thickness in a range of 4.4 to 5.4 *µ*m.

### TECHNICAL EFFECTS

According to the water sensor and the method for manufacturing the same according to the present disclosure, the protective layer made of PVB (polyvinyl butyral) such that hydrogen selectively passe through the protective layer while the protective layer blocks water may seal the color changeable layer and the catalyst layer. Thus, the sensor may sense hydrogen in both the liquid and the atmosphere.

### BRIEF DESCRIPTION OF DRAWINGS

FIG. 1 is a diagram for illustrating a hydrogen sensor according to an embodiment of the present disclosure.
FIG. 2A shows an image of a sensing result of hydrogen inside liquid by each of hydrogen sensors of Example 1 [glass substrate/color changeable layer (WO₃)/catalyst layer (Pd)/protective layer (PVB)] and Example 2 [PET substrate/color changeable layer (WO₃)/catalyst layer (Pd)/protective layer (PVB)]. FIG. 2B is a graph showing a result of measuring transmittance based on a wavelength before and after reaction of the hydrogen sensor of each of Examples 1 and 2 with hydrogen.
FIG. 3 shows an image after reaction of the hydrogen sensor of Example 2 with hydrogen and an image after maintaining the sensor for 10 seconds in an atmosphere free of hydrogen.
FIG. 4 is a graph showing a result of repeatedly performing a recovery process after reacting the hydrogen sensor of Example 2 with hydrogen.
FIG. 5 is an image for illustrating change in a thickness of a PVB-based protective layer based on the number of repetitions of a cycle of "a solution spin-coating process and a subsequent ultrapure water spin-coating process".
FIG. 6A shows images showing a result of immersion into water for 1 day of each of hydrogen sensors respectively having PVB-based protective layers formed by performing a cycle of "a solution spin-coating process and a subsequent ultrapure water spin-coating process" once, 5 times, and 7 times, respectively. FIG. 6B is a graph showing an amount of change in a color difference value before and after reaction of each of the hydrogen sensors in FIG. 6A with hydrogen in each of gaseous and liquid states.
FIG. 7A and FIG. 7B are graphs showing underwater durability and color change performance of each of hydrogen sensors respectively including protective layers made of PVB, PDMS (Polydimethylsiloxane), ZIF-8 (Zeolitic Imidazolate Framework), and Al₂O₃, respectively.

### DETAILED DESCRIPTIONS

The terminology used herein is for the purpose of describing particular embodiments only and is not intended to be limiting of the present disclosure. As used herein, the singular forms "a" and "an" are intended to include the plural forms as well, unless the context clearly indicates otherwise. It will be further understood that the terms "comprises", "comprising", "includes", and "including" when used in this specification, specify the presence of the stated features, integers, operations, elements, and/or components, but do not preclude the presence or addition of one or greater other features, integers, operations, elements, components, and/or portions thereof.

Unless otherwise defined, all terms including technical and scientific terms used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

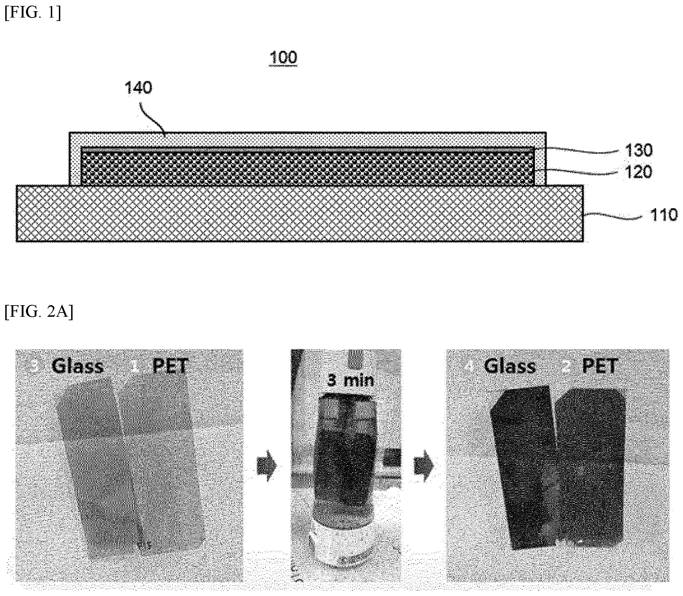

FIG. 1 is a diagram for illustrating a hydrogen sensor according to an embodiment of the present disclosure.

Referring to FIG. 1, a hydrogen sensor 100 according to an embodiment of the present disclosure includes a substrate 110, a color changeable layer 120, a catalyst layer 130, and a protective layer 140.

The substrate 110 may act as a support and may not be particularly limited as long as it may support the color changeable layer 120, the catalyst layer 130 and the protective layer 140. For example, the substrate 110 may be made of a material such as polymer, glass, ceramic, or metal.

The color changeable layer 120 may be disposed on the substrate 110 and made of an oxide semiconductor material that reacts with hydrogen ions or hydrogen atoms and thus changes a color thereof. In an embodiment, the color changeable layer 120 may be made of tungsten oxide (WO₃). The tungsten oxide changes its color under specific optical and chemical stimuli applied thereto. For example, when hydrogen ions or hydrogen atoms are supplied to the color changeable layer 120, the tungsten oxide reacts with the hydrogen ions or hydrogen atoms to form oxygen vacancies inside the color changeable layer 120, such that the tungsten oxide may absorb light in a near-infrared region, and a color of the tungsten oxide may change from dark gray to dark blue. In one example, when the reacted hydrogen ions or hydrogen atoms are desorbed from the tungsten oxide, a color of the color changeable layer 120 may be converted back to its initial color of the dark gray.

A method for forming the color changeable layer 120 is not particularly limited. The layer 120 may be formed on the substrate 110 using a known method of forming an oxide thinfilm. For example, the color changeable layer 120 may be formed on the substrate 110 using a vapor deposition method such as a sputtering process.

The catalyst layer 130 may be formed on the color changeable layer 120. The catalyst layer 130 may be made of a catalyst material that may dissociate hydrogen molecules (H₂) into hydrogen atoms (H) or hydrogen ions (H⁺). For example, the catalyst layer 130 may be made of palladium (Pd), platinum (Pt), or an alloy thereof.

The method of forming the catalyst layer 130 is not particularly limited. For example, the catalyst layer 130 may be formed via vapor deposition using an E-beam evaporator or the like.

In one embodiment, the catalyst layer 130 may be formed to have a thickness of about 4 to 4.5 nm. When the thickness of the catalyst layer 130 is smaller than 4 nm, the hydrogen dissociation reaction may be weakened, resulting in lower reactivity. When it exceeds 4.5 nm, a value of an initial transmittance of the sensor is lowered and thus an amount of change thereof during the hydrogen reaction is small. Thus, it may be difficult to obtain a sufficient signal.

The protective layer 140 may be formed to cover a surface of the catalyst layer 130 and an exposed surface of the color changeable layer 120. For example, the color changeable layer 120 and the catalyst layer 130 may be sealed with the substrate 110 and the protective layer 140.

In an embodiment, the protective layer 140 may be made of a polymer material which hydrogen molecules pass through while blocking water molecules. For example, the protective layer 140 may be made of PVB (polyvinyl butyral).

When the color changeable layer 120 and the catalyst layer 130 are exposed to moisture, the moisture may be adsorbed on the color changeable layer 120 and the catalyst layer 130. The adsorbed moisture may act as a barrier preventing contact of the hydrogen molecules with the metal catalyst of the catalyst layer 130, or may delay a rate of diffusion of the dissociated hydrogen atoms or hydrogen ions into the color changeable layer 120. Further, the tungsten oxide of the color changeable layer 120 may be converted into tungsten hydroxide (HWOₓ) due to various radical radicals inside the moisture. This may cause a problem in which the performance of the color changeable layer 120 is rapidly deteriorated. The protective layer 140 may allow the hydrogen molecules to pass through but may block water , thereby preventing external moisture from reaching the color changeable layer 120 and the catalyst layer 130. This may prevent the problem caused by the moisture as described above. Further, the protective layer 140 may allow the hydrogen sensor 100 according to an embodiment of the present disclosure to sense hydrogen in water.

In one embodiment, the protective layer 140 may be formed to have a thickness of about 4.4 to 5.4 µm. When the thickness of the protective layer 140 is smaller than 4.4 *µ*m, durability of the hydrogen sensor 100 may decrease, resulting in a problem that a lifetime thereof becomes too short. When it exceeds 5.4 *µ*m, the permeability of the hydrogen molecules through the protective layer is too low. Thus, there may be a problem that the sensing ability of hydrogen of the hydrogen sensor 100 is too low.

In an embodiment, the protective layer 140 may be formed using a solution process. For example, the protective layer 140 may be formed to cover a surface of the catalyst layer 130 and an exposed surface of the color changeable layer 120 using a spin-coating. Specifically, a cycle including a first step of forming a PVB film on the catalyst layer 130 via a spin coating process using a PVB solution containing methanol as a solvent and a second step of spin-coating ultrapure water (D.I water) on the PVB film may be performed a plurality of times to form the protective layer 140 having a preset thickness and waterproof characteristics. In this case, the thickness of the protective layer 140 may be adjusted based on the number of repetitions of the cycle including the first step and the second step.

According to the water sensor according to the present disclosure, the protective layer made of PVB (polyvinyl butyral) such that hydrogen selectively passe through the protective layer while the protective layer blocks water may seal the color changeable layer and the catalyst layer. Thus, the sensor may sense hydrogen in both the liquid and the atmosphere.

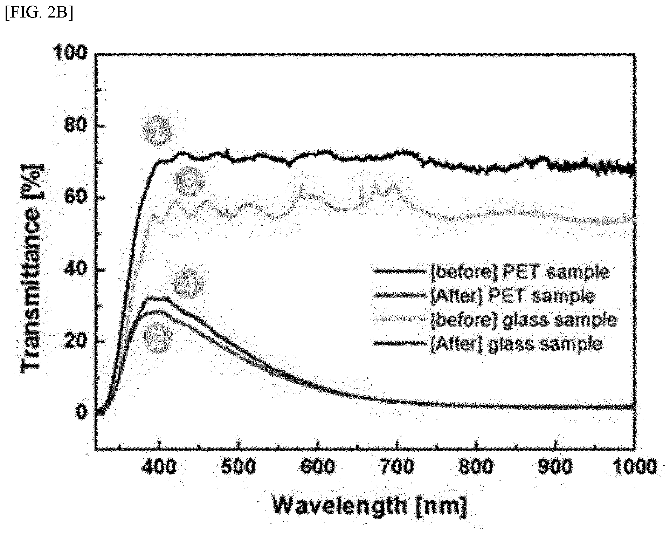

FIG. 2A shows an image of a sensing result of hydrogen inside liquid by each of hydrogen sensors of Example 1 [glass substrate/color changeable layer (WO₃)/catalyst layer (Pd)/protective layer (PVB)] and Example 2 [PET substrate/color changeable layer (WO₃)/catalyst layer (Pd)/protective layer (PVB)]. FIG. 2B is a graph showing a result of measuring transmittance based on a wavelength before and after reaction of the hydrogen sensor of each of Examples 1 and 2 with hydrogen.

Referring to FIG. 2A, when the hydrogen sensors of Examples 1 and 2 were immersed in hydrogen water for 3 minutes, it was identified that a color of each of the sensors was changed. That is, it may be identified that the hydrogen sensor of the present disclosure provided with the protective layer may stably detect hydrogen in the liquid.

Referring to FIG. 2B, the hydrogen sensor of Example 1 using a glass substrate had transmittance after reaction with hydrogen which was reduced by about 30 to 55% compared to transmittance before the reaction. The hydrogen sensor of Example 2 using a PET substrate had transmittance after reaction with hydrogen which decreased by about 40 to 70 compared to transmittance before the reaction.

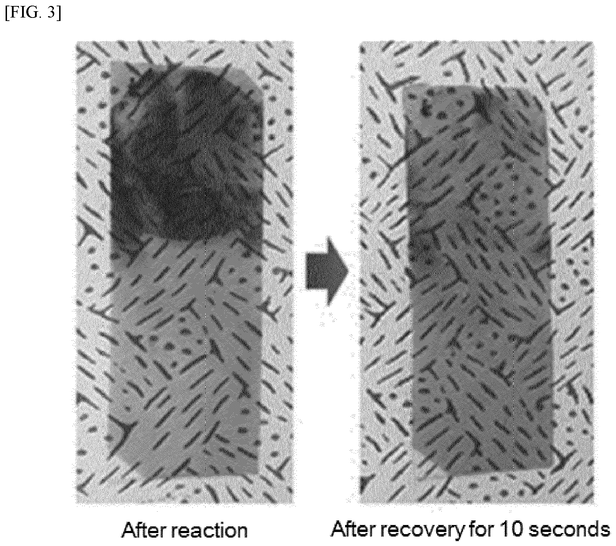

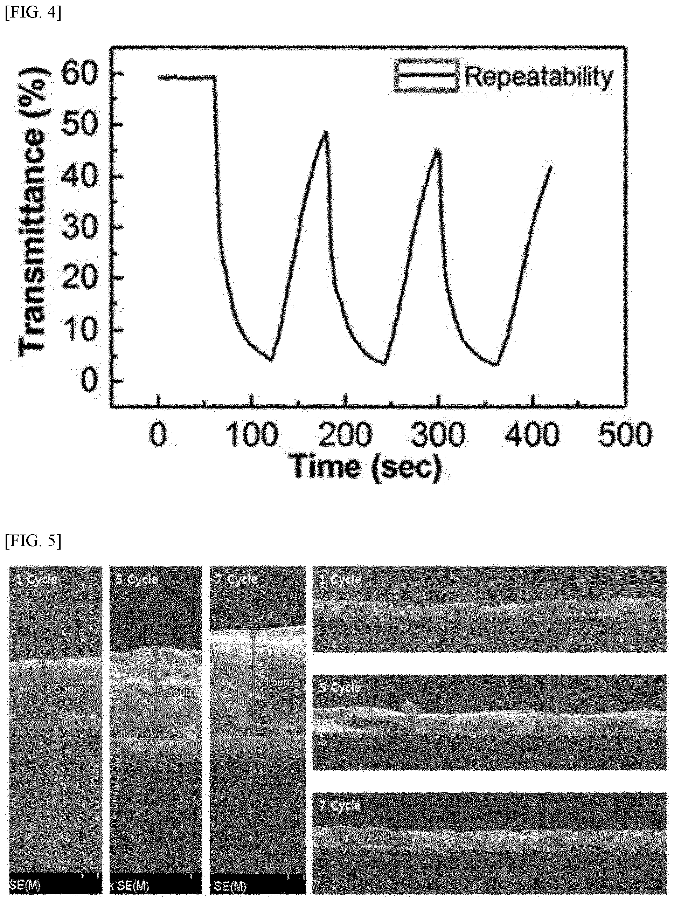

FIG. 3 shows an image after reaction of the hydrogen sensor of Example 2 with hydrogen and an image after maintaining the sensor for 10 seconds in an atmosphere free of hydrogen. FIG. 4 is a graph showing a result of repeatedly performing a recovery process after reacting the hydrogen sensor of Example 2 with hydrogen.

Referring to FIG. 3, it may be identified that when the hydrogen sensor of Example 2 which had a dark blue color after reaction with hydrogen for 1 minute is maintained in an atmosphere free of hydrogen, a color of the sensor is changed to the initial gray color again. That is, it may be identified that the hydrogen sensor according to the present disclosure may sense hydrogen repeatedly and has excellent characteristics such as a sensing speed (the sensor may sense the hydrogen within 1 minute) and a recovery speed (the sensor may be recovered within 10 seconds).

Referring to FIG. 4, it may be identified based on a result of repeatedly performing a cycle of reacting with hydrogen and recovering the sensor that the hydrogen sensor of Example 2 may sense hydrogen stably.

FIG. 5 is an image for illustrating change in a thickness of a PVB-based protective layer based on the number of repetitions of a cycle of "a solution spin-coating process and a subsequent ultrapure water spin-coating process".

Referring to FIG. 5, when one cycle of "a solution spin-coating process and a subsequent ultrapure water spin-coating process" was performed, the protective layer made of PVB and having a thickness of about 3.53 *µ*m was formed. When 5 cycles were performed, the PVB-based protective layer with a thickness of about 5.36 µm was formed. When 7 cycles were performed, the PVB-based protective layer with a thickness of about 6.15 µm was formed. That is, it may be identified that the thickness of the PVB-based protective layer almost linearly increases as the number of repetitions of the cycle of "a solution spin-coating process and a subsequent ultrapure water spin-coating process" increases. As a result, it may be identified that the thickness of the PVB-based protective layer may be controlled based on the number of repetitions of the cycle of "a solution spin-coating process and a subsequent ultrapure water spin-coating process".

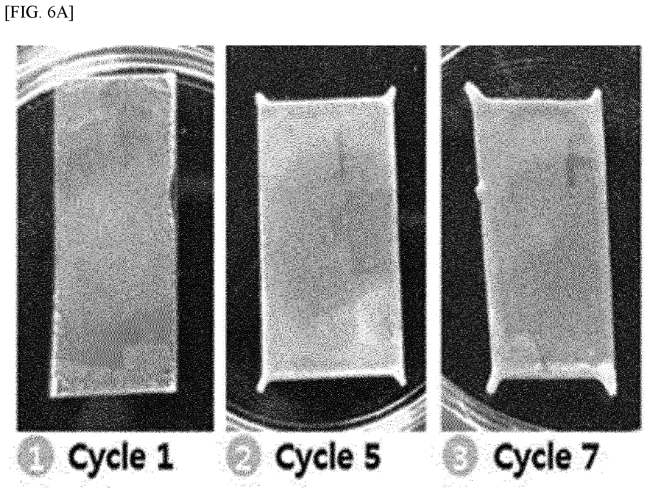

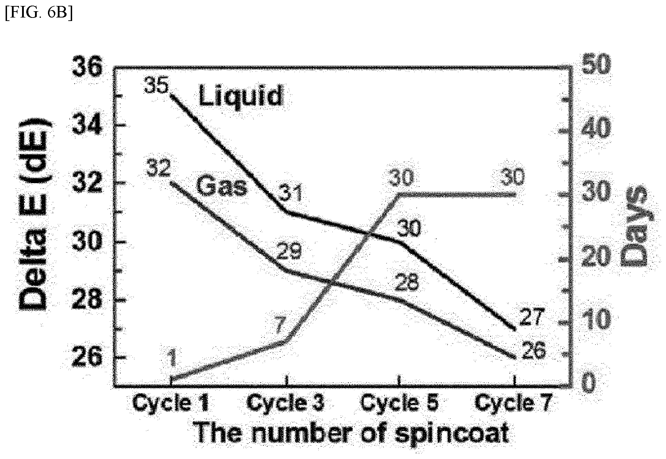

FIG. 6A shows images showing a result of immersion into water for 1 day of each of hydrogen sensors respectively having PVB-based protective layers formed by performing a cycle of "a solution spin-coating process and a subsequent ultrapure water spin-coating process" once, 5 times, and 7 times, respectively. FIG. 6B is a graph showing an amount of change in a color difference value before and after reaction of each of the hydrogen sensors in FIG. 6A with hydrogen in each of gaseous and liquid states.

Referring to FIG. 6A, it was identified that when a hydrogen sensor having the protective layer formed by performing one cycle of "a solution spin-coating process and a subsequent ultrapure water spin-coating process" was immersed in water for 1 day, the protective layer was detached, and thus durability of the sensor was not good. In contrast, it was identified that when each of hydrogen sensors having the protective layers formed by performing the cycle of "a solution spin coating process and subsequent ultrapure water spin-coating process" 5 and 7 times, respectively was immersed in water for 1 month, the protective layer was stably maintained and thus each sensor had excellent durability. Therefore, preferably, the protective layer may have a thickness larger than 3.53 *µ*m thickness achieved when the cycle of "a solution spin-coating process and a subsequent ultrapure water spin-coating process" is performed once. In other words, the protective layer may have a thickness of about 4.4 µm or larger achieved when the cycle of "a solution spin-coating process and a subsequent ultrapure water spin-coating process" is performed about 3 times or greater.

Referring to FIG. 6B, it was identified that as the thickness of the protective layer increased, an amount of change in a color difference value before and after reaction of the sensor with hydrogen in both gaseous and liquid states decreased. In particular, when the protective layer was formed by performing the cycle of "a solution spin-coating process and a subsequent ultrapure water spin-coating process" 3 to 5 times, the change in the color difference value was relatively smaller compared to other cases. Therefore, the protective layer is preferably formed to have a thickness in a range of about 4.4 to 5.4 µm, as achieved when the cycle of "a solution spin-coating process and a subsequent ultrapure water spin-coating process" is performed 3 to 5 times.

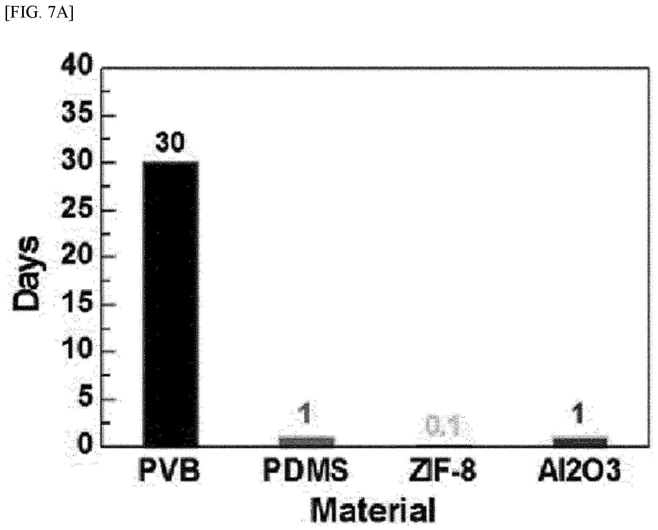

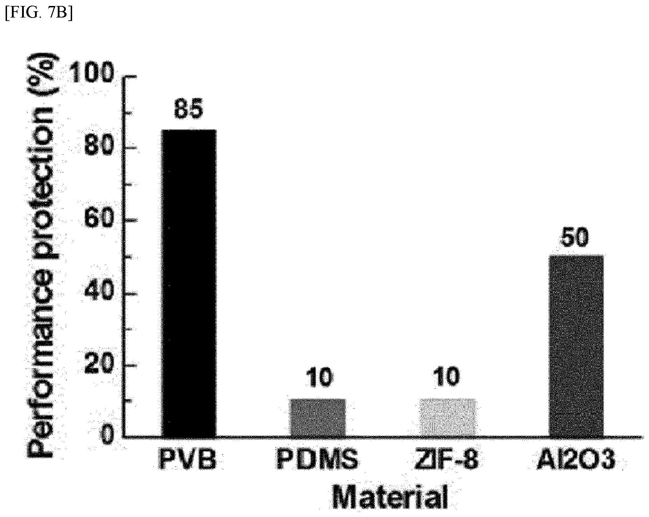

FIG. 7A and FIG. 7B are graphs showing underwater durability and color change performance of each of hydrogen sensors respectively including protective layers made of PVB, PDMS (Polydimethylsiloxane), ZIF-8 (Zeolitic Imidazolate Framework), and Al₂O₃, respectively. The graph of FIG. 7B shows a percentage of a color change performance of the present sensor relative to a color change performance of a hydrogen sensor free of the protective layer.

Referring to FIG. 7A, it was identified that the PVB-based protective layer stably protected the hydrogen sensor from moisture in the hydrogen water and the sensor maintained its performance for 30 days, whereas each of a PDMS-based protective layer, a ZIF-8-based protective layer, and a Al₂O₃-based protective layer was not able to maintain the performance of the sensor for more than 1 day.

Referring to FIG. 7B, the sensor to which the PVB-based protective layer was applied exhibited 85% of the color change performance in the hydrogen water, relative to the color change performance of the hydrogen sensor free of the protective layer. Each of the sensors to which a PDMS-based protective layer and a ZIF-8-based protective layer were applied respectively exhibited about 10% of the color change performance, relative to the color change performance of the hydrogen sensor free of the protective layer. The sensor to which the Al₂O₃-based protective layer was applied exhibited 50% of the color change performance in the hydrogen water, relative to the color change performance of the hydrogen sensor free of the protective layer.

Therefore, it may be identified that the sensor in which the protective layer is made of PVB may have improved durability and color change performance in water. This is because the PVB-based protective film may allow the hydrogen molecules or ions smaller than the water molecules to pass therethrough but may block the water molecules.

Although the disclosure has been described above with reference to the preferred embodiments of the present disclosure, those skilled in the art will understand that various modifications and changes may be made to the present disclosure without departing from the spirit and scope of the present disclosure as set forth in the following claims.

### Refereence numerals

100: Hydrogen sensor 110: Substrate
120: Color changeable layer 130: Catalyst layer
140: Protective layer

## Claims

1. A hydrogen sensor comprising:
a substrate;
a color changeable layer disposed on the substrate and made of an oxide semiconductor material, wherein when the oxide semiconductor material reacts with hydrogen ions or hydrogen atoms, a color thereof changes;
a catalyst layer disposed on a surface of the color changeable layer and made of a catalyst material, wherein the catalyst material dissociates hydrogen molecules (H₂) into hydrogen atoms (H) or hydrogen ions (H⁺); and
a protective layer disposed to cover a surface of the catalyst layer and an exposed surface of the color changeable layer, and made of a polymer material, wherein the polymer material allows hydrogen molecules to pass therethrough but blocks water molecules.

2. The hydrogen sensor of claim 1, wherein the catalyst layer is made of palladium (Pd), platinum (Pt) or an alloy containing at least one thereof.

3. The hydrogen sensor of claim 2, wherein the catalyst layer is formed to have a thickness in a range of 4 to 4.5nm.

4. The hydrogen sensor of claim 1, wherein the color changeable layer and the catalyst layer are sealed with the substrate and the protective layer.

5. The hydrogen sensor of claim 1, wherein the protective layer is made of PVB (polyvinyl butyral).

6. The hydrogen sensor of claim 5, wherein the protective layer is formed to have a thickness in a range of 4.4 to 5.4µm.

7. A method for manufacturing a hydrogen sensor, the method comprising:
depositing tungsten oxide on a substrate to form a color changeable layer;
depositing palladium or platinum on the color changeable layer to form a catalyst layer; and
performing a cycle 4 to 6 times to form a protective layer covering a surface of the catalyst layer and an exposed surface of the color changeable layer, wherein the cycle is composed of a first step of forming a PVB film on the catalyst layer using a spin coating process of a PVB (polyvinyl butyral) solution and a second step of spin-coating ultrapure water on the PVB film.

8. The method of claim 7, wherein the protective layer is formed to have a thickness in a range of 4.4 to 5.4 *µ*m.
